# EUROPEAN PATENT APPLICATION

(11) **EP 2 959 920 A1**
(43) Date of publication of application: **30.12.2015**
(21) Application number: 15174028.9
(22) Date of filing: 26.06.2015
(51) Int. Cl.: A61L 9/03, A61L 9/12, A61L 9/14, H01R 13/00

(54) **AROMA DELIVERY DEVICE**

(30) Priority: 27.06.2014 IT UD20140111
(71) Applicant: GISA S.R.L., 28068 Romentino NO (IT)
(72) Inventor: Guaglio, Alessandro, 28069 Trecate (NO) (IT)
(74) Representative: Petraz, Gilberto Luigi

(57) **Abstract**

Aroma delivery device comprising a containing body (12) which houses at least a tank (13) containing an aromatic substance and which is provided with an aromatic substance delivery head (15). The aroma delivery device also comprises an activator electronic circuit (16) configured to cause the delivery of the aromatic substance contained in said tank (13) through the delivery head (15). The delivery device comprises a USB connector (18) associated with the containing body (12) and connected to the activator electronic circuit (16) to provide electric power to the latter.

The containing body (12) is defined by a first portion (20) that develops mainly along a first axis (X) and is provided with a first end (38) with which the USB connector (18) is associated, and by a second portion (21) that develops mainly along a second axis (Y), transverse with respect to the first axis (X), and is provided with a second end (39) with which the delivery head (15) is associated.

## Description

### FIELD OF THE INVENTION

The present invention concerns an aroma delivery device powered electrically by means of a USB (Universal Serial Bus) connector, by an electronic device such as a computer, television, cigarette lighter or similar or comparable devices provided with USB connection ports.

### BACKGROUND OF THE INVENTION

Aroma delivery devices are known, configured to release into the air aromatic vapors produced by an activator electronic circuit, like an ultrasound mechanism.

The ultrasound mechanism ionizes the aromatic substance by vibrations and transforms it into vapor that is nebulized into the environment.

The aroma delivery device is also provided with a containing body with a substantially elongated shape in a single main direction, hollow inside and in which the activator electronic circuit is installed, and a tank containing the aromatic substance.

The aroma delivery device is also provided with a USB connector attached protruding from the containing body and connectable to the electric power device, such as a computer or television; the latter is provided in turn with a complementary USB port that, when connected to the USB connector, powers the activator electronic circuit electrically.

The containing body is provided with a delivery head that puts the containing tank into communication with the outside, allowing to release the aromatic vapors into the air.

The delivery head is usually located in a wall, upper during use, of the containing body or on a lateral wall with respect to the USB connector.

This position entails a non-uniform, not well-distributed diffusion of the vapors into the environment, and the aroma remains confined, for example, in proximity to the diffusion zone, that is, in proximity to the electric power device.

If the delivery device is connected to a computer, for example of the portable type and used by a user, he/she can be affected negatively by the presence of high concentrations of aromatic vapor in correspondence with the work station, due to the poor diffusion of the vapor into the air. In fact, the particular position of the USB ports of the computer is such that, when the USB connector is connected to the latter, it typically puts the delivery head in a position interfering for example with the support plane of the computer itself.

One purpose of the present invention is to obtain an aroma delivery device that optimizes the diffusion of the vapors into the air.

Another purpose of the present invention is to obtain an aroma delivery device that is simple and economical.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, the present invention concerns an aroma delivery device that comprises:
- a containing body which houses at least a tank containing an aromatic substance and which is provided with an aromatic substance delivery head;
- an activator electronic circuit configured to cause the delivery of the aromatic substance contained in the tank through the delivery head;
- a USB connector associated with the containing body and connected to the activator electronic circuit to provide electric power to the latter.

According to one aspect of the present invention, the containing body is defined by a first portion that develops mainly along a first axis and is provided with a first end with which the USB connector is associated, and by a second portion that develops mainly along a second axis, transverse with respect to the first axis, and is provided with a second end with which the delivery head is associated.

The development of the delivery device along the first and second axis allows to dispose the delivery head in a position that promotes a homogeneous and well-distributed diffusion of the aromatic substance with respect to the electronic device to which the USB connector is connected.

Moreover, the position of the delivery head, when the delivery device is applied to portable computers, facilitates the delivery of the aromatic substance in a direction that does not involve the user directly.

According to a possible solution, the first axis and the second axis can be reciprocally angled with respect to each other by an angle of amplitude comprised between 90° and 175°, preferably between 110° and 160°. These angles between the axes increase the efficiency of distribution of the aromatic vapors.

According to another form of embodiment, the first axis and the second axis lie on the same lying plane. This allows to limit the bulk of the delivery device also in relation to the electronic device to which it will be connected.

In variant forms of embodiment of the delivery device, it is provided that it comprises a memory card, contained in the containing body and that allows to store data. This increases the versatility of the delivery device.

In another variant form of embodiment, the first portion and second portion are angularly positionable with respect to each other, for example by articulation elements, to allow an optimum positioning of the delivery head with respect to the USB connector.

In another variant form of embodiment, the USB connector is retractable in the containing body, to limit the bulk and to prevent accidental damage thereto.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a perspective view of the aroma delivery electronic device according to the present invention;
- fig. 2 is a section view of fig. 1.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF SOME FORMS OF EMBODIMENT

We shall now refer in detail to the various forms of embodiment of the present invention, of which one or more examples are shown in the attached drawings. Each example is supplied by way of illustration of the invention and shall not be understood as a limitation thereof. For example, the characteristics shown or described insomuch as they are part of one form of embodiment can be adopted on, or in association with, other forms of embodiment. It is understood that the present invention shall include all such modifications and variants.

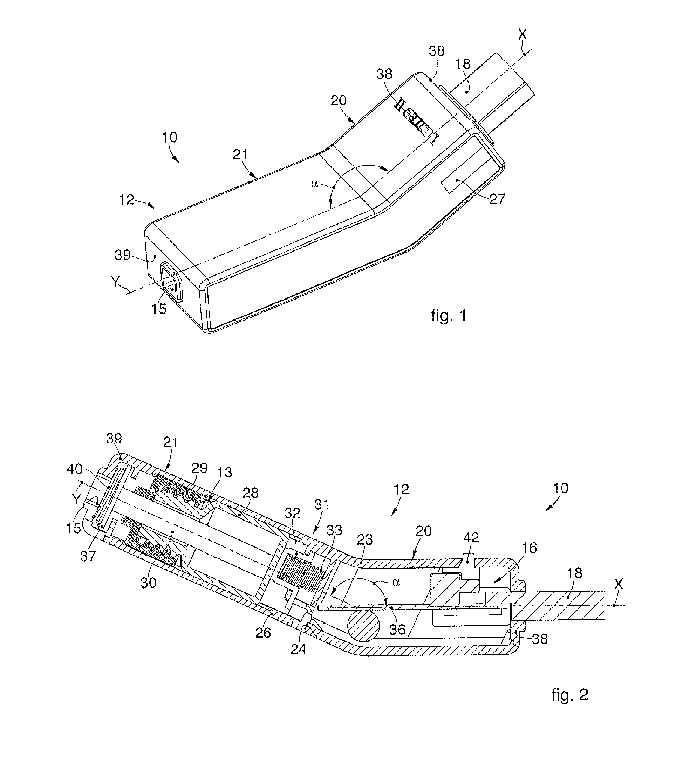

Figs. 1-2 are used to describe forms of embodiment of an aroma delivery device 10 configured to release into the air the vapors of an aromatic substance, such as for example an essential oil.

The delivery device 10 comprises a containing body 12 in which a tank 13 for containing the aromatic substance is positionable.

The containing body 12 is provided with a delivery head 15 to deliver the aromatic substance into the air, which puts the tank 13 into communication with the outside, allowing it to be dispersed into the environment.

An activator electronic circuit 16 is positioned in the containing body 12, and is put in cooperation with the tank 13 to determine the delivery of the aromatic substance.

A USB connector 18 is also associated with the containing body 12, which is connected to the activator electronic circuit 16 and which can be selectively connectable to an electronic device to supply electric power to the USB connector 18 and consequently to the activator electronic circuit 16.

The containing body 12 is defined by a first portion 20 that develops mainly along a first axis X and a second portion 21 that develops mainly along a second axis Y, transverse to the first axis X.

The first portion 20 and the second portion 21 can be provided, respectively, with a first end 38 and a second end 39, opposite each other, which delimit the longitudinal development of the containing body 12.

The USB connector 18 is associated with the first end 38, and the delivery head 15 is associated with the second end 39.

The second end 39 can be provided with a front wall opposite the USB connector 18 on which the delivery head 15 can be present. The front wall is disposed incident to the second axis Y.

According to the form of embodiment in figs. 1 and 2, the first portion 20 and the second portion 21 are at least partly made in a single body with each other.

According to a possible variant form of embodiment, between the first portion 20 and the second portion 21 articulation elements can be provided to modify the reciprocal angle of the first axis X and the second axis Y. The articulation elements can have one or more degrees of freedom to determine the possible orientations of the first portion 20 with respect to the second portion 21.

The first axis X and the second axis Y can lie on the same lying plane π. This allows to contain the overall bulk of the delivery device 10.

Merely by way of example, the first axis X and the second axis Y can be reciprocally angled with respect to each other by an angle α with an amplitude comprised between 90° and 175°, preferably between 110° and 160°.

This latter range of values allows to optimize the distribution of the aromatic substance in the environment.

The containing body 12 can be defined by a first container 23 and a second container 24, connected to each other by removable attachment elements such as snap-in connection elements, clips, joints or geometric interference.

In some forms of embodiment, see fig. 2 for example, the first container 23 can affect the substantial part of the containing body 12, while the second container 24 can be only a part, for example the lower part, of the containing body 12.

At least one of either the first container 23 or the second container 24 can be provided with an aperture 25 to allow to introduce or remove the containing tank 13 into/from the containing body 12.

The aperture 25 can be selectively opened and closed by a closing element 26.

With reference to figs. 1-2, the aperture 25 can be made in the second portion 21 of the containing body 12.

The tank 13 can be positioned inside the containing body 12 and in its second portion 21.

The tank 13 can comprise a bottle 28 configured to contain the aromatic substance and a delivery element 30 configured to take the aromatic substance from the bottle 28 and allow to deliver it into the air by the delivery head 15.

The delivery element 30 can protrude from the containing tank 13 and can be elongated in shape, like a wick.

The protruding position of the delivery element 30 allows to dispose the activator electronic circuit 16 in cooperation with the delivery element 30 in order to determine the diffusion of the aromatic substance.

The tank 13 comprises a stopper 29 connected to the bottle 28 to prevent leakage of the aromatic substance from the bottle 28.

The stopper 29 can be provided with a through aperture parallel to the second axis Y and configured to hold the delivery element 30 in the bottle 28.

The delivery head 15 can comprise a hole, through in the thickness of the front wall of the second end 39 of the containing body 12.

The delivery head 15 can be aligned with the delivery element 30 along the second axis Y.

The delivery head 15 can be made in a single body with the containing body 12 or it can be a separate element.

The delivery head 15 can be integrated in the bulk of the containing body 12, see figs. 1-2 for example, or can protrude from it.

The activator electronic circuit 16 can be selectively connected by the USB connector 18 to an electronic device from which the activator electronic circuit 16 takes the electric power needed to deliver the aromatic substance.

The activator electronic circuit 16 comprises an electronic board 36 connected to the USB connector 18 and contained in the bulk of the containing body 12.

The activator electronic circuit 16 can also comprise an exciter 37 configured to cause said aromatic substance to evaporate. To this purpose, during use, the exciter 37 can be put in direct contact with the delivery element 30 of the tank 13.

The electronic board 36 and the exciter 37 can be connected to each other by connection wires, not shown in figs. 1-2.

In some forms of embodiment, the exciter 37 can comprise an ultrasound mechanism to emit ultrasounds toward the tank 13 and which is associated with the containing body 12.

The exciter 37 can be positioned near the second end 39 of the containing body 12, in particular, it can be interposed between the delivery head 15 and the delivery element 30.

The exciter 37 is kept resting against the delivery element 30 by an elastic element 40, such as for example a spring.

The electronic board 36 allows to power the exciter 37 with suitable electric parameters, converting and/or remodulating the electric power supplied by the electronic device to which the delivery device 10 is connected.

The containing body 12 also comprises a presser member 31 provided to guarantee the reciprocal contact between the tank 13, in this case its delivery element 30, and the exciter 37 of the activator electronic circuit 16.

The presser member 31 comprises a pressing element 32 with which an elastic element 33 is associated, such as a spring, to thrust the pressing element 32 against the tank 13.

In some forms of embodiment, the activator electronic circuit 16 can also comprise a switch 42, configured to selectively activate the exciter 37.

In some forms of embodiment, the switch 42 comprises a selector able to set the delivery cycles of the aromatic substance by the activator electronic circuit 16.

The selector can be connected for example to a timer of the electronic board 36, and the position of the selector determines the frequency with which the aromatic substance is delivered.

According to a variant form of embodiment, the switch 42 can be configured to allow to adjust the quantity of aromatic substance delivered and consequently the degree of diffusion of the perfume into the environment.

In some forms of embodiment, see figs. 1-2 for example, the switch 42 can be positioned in the first portion 20 of the containing body 12.

The switch 42 can also be disposed outside the containing body 12, to facilitate activation thereof by a user.

The USB connector 18 can be associated with the containing body 12 protruding therefrom.

With reference to figs. 1-2, the USB connector 18 can have a longitudinal development along the second axis Y, to be connectable to the electronic device having a complementary port.

According to a variant form of embodiment, the USB connector 18 can be retractable in the bulk of the containing body 12, to improve the management of the spaces therein.

The USB connector 18 can be a substantially known type, and can be chosen from the group comprising A type or B type USB connectors, A type or B type mini-USB, A type or B type micro-USB.

In some forms of embodiment, the delivery device 10 can comprise a memory card for storing data, such as documents, photos, films. The memory card can be disposed inside the containing body 12 and connected to the USB connector 18, to allow data exchange.

In some forms of embodiment, see fig. 1 for example, the containing body 12 can be provided with at least one luminous element 27, such as for example a led, to identify an active or inactive condition of the activator electronic circuit 16.

The luminous element 27 supplies a visual indication of the functioning condition of the delivery device 10, using a color code for example.

It is clear that modifications and/or additions of parts may be made to the delivery device as described heretofore, without departing from the field and scope of the present invention.

It is also clear that, although the present invention has been described with reference to some specific examples, a person of skill in the art shall certainly be able to achieve many other equivalent forms of electronic aroma delivery device, having the characteristics as set forth in the claims and hence all coming within the field of protection defined thereby.

## Claims

1. Aroma delivery device comprising:
- a containing body (12) which houses at least a tank (13) containing an aromatic substance and which is provided with an aromatic substance delivery head (15);
- an activator electronic circuit (16) configured to cause the delivery of the aromatic substance contained in said tank (13) through said delivery head (15);
- a USB connector (18) associated with said containing body (12) and connected to said activator electronic circuit (16) to provide electric power to the latter, **characterized in that** said containing body (12) is defined by a first portion (20) that develops mainly along a first axis (X) and is provided with a first end (38) with which said USB connector (18) is associated, and by a second portion (21) that develops mainly along a second axis (Y), transverse with respect to said first axis (X), and is provided with a second end (39) with which said delivery head (15) is associated.

2. Delivery device as in claim 1, **characterized in that** said second end (39) is provided with a front wall opposite to said USB connector (18), said delivery head (15) being on said front wall.

3. Delivery device as in any claim hereinbefore, **characterized in that** said first axis (X) and said second axis (Y) are reciprocally angled with respect to each other by an angle (α), with an amplitude comprised between 90° and 175°, preferably between 110° and 160°.

4. Delivery device as in any claim hereinbefore, **characterized in that** said first axis (X) and said second axis (Y) lie on the same lying plane (π).

5. Delivery device as in any claim hereinbefore, **characterized in that** said first portion (20) and said second portion (21) are angularly positionable with respect to each other by articulation elements.

6. Delivery device as in any claim hereinbefore, **characterized in that** said tank (13) is positioned, inside said containing body (12), in said second portion (21) thereof.

7. Delivery device as in any claim hereinbefore, **characterized in that** said activator electronic circuit (16) comprises an electronic board (36) connected to said USB connector (18) and contained in the bulk of said containing body (12), and an exciter (37) put in cooperation with said tank (13) and configured to cause said aromatic substance to evaporate.

8. Delivery device as in claim 7, **characterized in that** said activator electronic circuit (16) comprises a switch (42) configured to selectively activate said exciter (37).

9. Delivery device as in any claim hereinbefore, **characterized in that** said USB connector (18) is selectively retractable into the bulk of said containing body (12).

10. Delivery device as in any claim hereinbefore, **characterized in that** it comprises a memory card configured to store data disposed inside said containing body (12).
